# EUROPEAN PATENT APPLICATION

(11) **EP 3 103 505 A1**
(43) Date of publication of application: **14.12.2016**
(21) Application number: 15171789.9
(22) Date of filing: 12.06.2015
(51) Int. Cl.: A61M 39/10, A61M 5/142

(54) **JOINING UNIT FOR DETECTING NEEDLE DISCONNECTION**

(71) Applicant: Nokia Technologies Oy, 02610 Espoo (FI)
(72) Inventor: Petäinen, Katriina, 36200 Kangasala (FI); Naskali, Matti, Tokyo, Tokyo 158-0084 (JP)
(74) Representative: Espatent Oy

(57) **Abstract**

A joining unit (400) for controlling a connection between an infusion pump (200) and a cannula (310) is described. The joining unit has a connection detector for detecting whether an infusion pump is connected to a cannula. The joining unit further has a wireless transmitter for transmitting to the infusion pump a connection indication in response to detecting by the connection detector that the infusion pump is connected to the cannula.

## Description

### TECHNICAL FIELD

The present application generally relates to infusion therapy control.

### BACKGROUND

This section illustrates useful background information without admission of any technique described herein representative of the state of the art.

Infusion therapy generally refers to administering medication through a cannula to a human or animal body. For example, diabetes is often treated with insulin infusion therapy using infusion pumping to maintain desired administering of medical substance. Insulin pumps used by diabetics usually contain an insulin pump and an infusion set that is connected to the insulin pump and that serves to deliver insulin under the skin through a cannula of the infusion set. Insulin pumps have greatly helped diabetics to avoid potentially sever consequences of insufficient insulin treatment by reducing the need to remember inject new insulin doses and they also help people with needle phobia as it suffices to penetrate the skin of a diabetic only once in two or three days instead doing so many times a day.

The infusion set is normally manufactured so that the insulin pump is detachable and again attachable to the cannula by the user. For example, the insulin pump can be disconnected before bathing or for some maintenance operations of the infusion pump so as to protect the pump or avoid safety hazards. To enable a releasable connection, some insulin pumps are connected to the cannula via a connector mounted in tubing (hose) between the insulin pump and the cannula, e.g. a few centimeters upstream to the cannula. The connector and/or cannula has a sealing in order to avoid entry of air, water or dirt into the infusion set and thereby under the skin. On reconnecting the insulin pump a fluid connection is reopened between the insulin pump and the connector and/or cannula.

It is desired to provide new and/or more robust techniques for monitoring connection status of a user releasable connection between infusion pump and an infusion set.

### SUMMARY

Various aspects of examples of the invention are set out in the claims.

According to a first example aspect of the present invention, there is provided a joining unit comprising:
a connection detector configured to detect whether an infusion pump is connected to a cannula; and
a wireless transmitter configured to transmit to the infusion pump a connection indication in response to detecting by the connection detector that the infusion pump is connected to the cannula.

The joining unit may comprise a connector configured to user releasably connect the infusion pump to the cannula into a fluid connection. The term fluid connection refers to connection that enables desired flow of an infusion fluid. The connector may comprise a cannula connection port for connecting to the cannula for arranging a fluid connection with the cannula. The connector may comprise an infusion pump connection port for connecting to the infusion pump for arranging a fluid connection with the infusion pump. The infusion pump connection port may be releasably connectable by a user to an infusion output of the infusion pump. The infusion pump connection port may be connectable to the output via a flexible tubing. The connector may comprise a form lock configured to user releasably connect the connector to the cannula. The form lock may comprise a bayonet base or a bayonet base socket. The form lock may comprise a thread. The connector may be configured to enable the connection detector to detect from the connector whether the connector is connected to the cannula. The connector may be configured to inhibit the connection detector from detect that the connector is connected to the cannula unless the connector is in the fluid connection with the cannula.

The infusion pump may be configured to administer insulin. The infusion pump may be configured to administer pain medication. The infusion pump may be an ambulatory infusion pump.

The wireless transmitter may comprise a radio transmitter. Alternatively, or additionally, the wireless transmitter may comprise an ultrasound transmitter. The ultrasound transmitter may be configured to employ at least one of the tubing and the infusion fluid to convey ultrasonic signals for the infusion pump. The term wireless may refer to the lack of electric wires.

The joining unit may comprise a housing. The housing may be water-proof. The housing may be hermetic. The housing may be water-resistant. The housing may contain the wireless transmitter. The housing may contain the connection detector. The housing may comprise a wall separating the connection detector from the connector. The connection detector may be configured to detect the connection through the wall. The wall may be flexible. The connection detector may be configured to detect the connection from flexing of the wall. The form lock may be configured to flex the wall when the connector is connected to or disconnected from the cannula. The housing may contain a battery for operating the wireless transmitter. The connection detector may be configured to power the wireless transmitter using the battery when the connection detector detects that the connector is connected to the cannula.

The wireless transmitter may be configured to periodically transmit connection indications in response to detecting by the connection detector that the infusion pump is connected to the cannula.

The joining unit may comprise a receiver configured to receive an infusion interval setting from an infusion pump unit that comprises the infusion pump. The receiver may be a wireless receiver. The joining unit may be configured to adjust the periodic transmitting of the connection indications according to a pumping interval of the infusion pump.

The joining unit may comprise an input configured to receive feedback from a sensor. The sensor may be a glucose sensor. The transmitter may be configured to transmit the feedback for the infusion pump for adjustment of infusion therapy adhered by the infusion pump. The sensor may be comprised by a cannula set that comprises the cannula.

According to a second example aspect of the present invention, there is provided a system comprising the joining unit of the first example aspect. The system may comprise the infusion pump. The system may comprise the cannula configured to deliver infusion into subcutaneous tissue of a user of the system. The system may comprise a cannula set that comprises the cannula. The cannula set may comprise a skin attachment member configured to maintain the cannula in place for at least a plurality of hours. The cannula may comprise a connector port configured to form a fluid connection with the infusion pump through the connector. The cannula set may be disposable. The system may comprise a tubing for interconnecting the infusion pump and the cannula. The tubing may be disposable. The system may comprise a battery for operating the wireless transmitter. The battery may be disposable. The battery may be rechargeable. The system may comprise the sensor.

According to a third example aspect of the present invention, there is provided a method comprising:
detecting whether an infusion pump is connected to a cannula; and
wirelessly transmitting to the infusion pump a connection indication in response to detecting that the infusion pump is connected to the cannula.

According to a fourth example aspect of the present invention, there is provided a computer program comprising computer executable program code configured to execute any method of the third example aspect.

The computer program may be stored in a computer readable memory medium.

Any foregoing memory medium may comprise a digital data storage such as a data disc or diskette, optical storage, magnetic storage, holographic storage, opto-magnetic storage, phase-change memory, resistive random access memory, magnetic random access memory, solid-electrolyte memory, ferroelectric random access memory, organic memory or polymer memory. The memory medium may be formed into a device without other substantial functions than storing memory or it may be formed as part of a device with other functions, including but not limited to a memory of a computer, a chip set, and a sub assembly of an electronic device.

According to a fifth example aspect of the present invention, there is provided a joining unit comprising a memory and a processor that are configured to cause the joining unit to perform the method of the second example aspect.

According to a sixth example aspect of the present invention, there is provided a joining unit comprising means for performing the method of the second example aspect.

Different non-binding example aspects and embodiments of the present invention have been illustrated in the foregoing. The embodiments in the foregoing are used merely to explain selected aspects or steps that may be utilized in implementations of the present invention. Some embodiments may be presented only with reference to certain example aspects of the invention. It should be appreciated that corresponding embodiments may apply to other example aspects as well.

### BRIEF DESCRIPTION OF THE DRAWINGS

For a more complete understanding of example embodiments of the present invention, reference is now made to the following descriptions taken in connection with the accompanying drawings in which:
Fig. 1 shows an architectural drawing of a system of an example embodiment;
Fig. 2 shows a block diagram of an infusion pump unit of an example embodiment;
Fig. 3 shows a schematic drawing of a tubing, a cannula set and a joining unit of an example embodiment;
Fig. 4 shows a block diagram of the joining unit of an example embodiment;
Fig. 5 shows a schematic block diagram of a connection detector of an example embodiment; and
Fig. 6 shows a flow chart of a process according to an example embodiment.

### DETAILED DESCRIPTON OF THE DRAWINGS

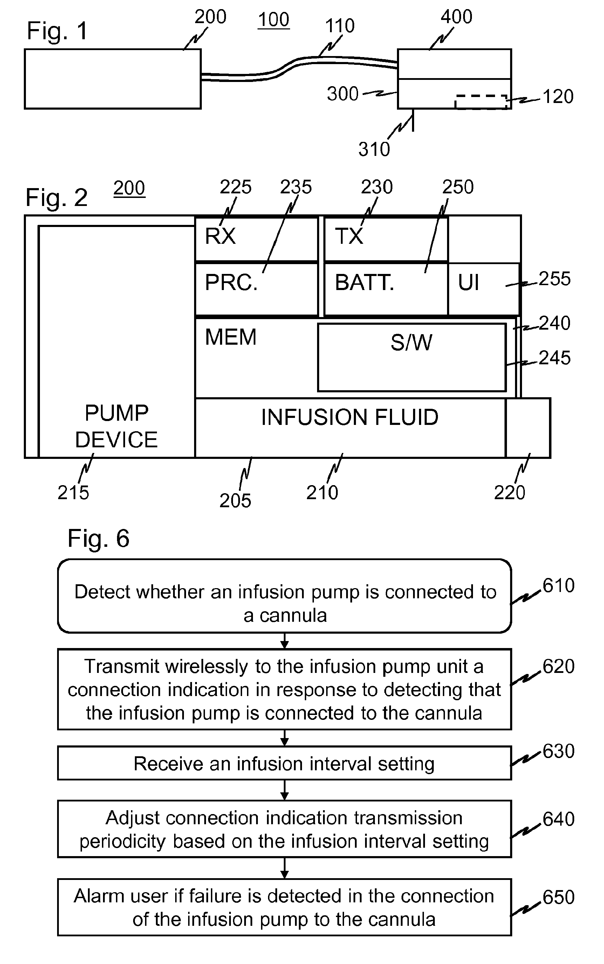

An example embodiment of the present invention and its potential advantages are understood by referring to Figs. 1 through 6 of the drawings. In this document, like reference signs denote like parts or steps.

Fig. 1 shows an architectural drawing of an infusion fluid delivery system 100 of an example embodiment. The system 100 comprises an infusion pump unit 200 that comprises an infusion pump or infusion pump device 215 (Fig. 2), a cannula set 300 and a joining unit 400 for interconnecting the infusion pump unit 200 (or more accurately, the infusion pump device 215) and the cannula set 300 in a fluid connection, and a tubing 110 for interconnecting the joining unit 400 and the pump unit 200. In an example embodiment, the system 100 comprises a monitoring system for monitoring the status of the user wearing the cannula set (or patient). The infusion fluid delivery system may be an insulin delivery system, insulin pump. The insulin pump may comprise a continuous glucose monitoring (CGM) system 120 integrated to the cannula set.

Fig. 2 shows a block diagram of the infusion pump unit 200 of an example embodiment. The infusion pump unit 200 comprises an infusion fluid storage 205 comprising infusion fluid 210, an output port 220 for connecting with the tubing 110, the infusion pump device (or dosing device) 215 configured to deliver a dose of set amount (e.g. volume or mass) of the infusion fluid 210 to the tubing 110 via the output port for delivery to the cannula set 300 through the joining unit 400, a wireless receiver 225, a transmitter 230, a processor 235 configured to control operation of the infusion pump unit, a memory 240 comprising computer program code 245 for controlling the processor, and a battery 250 for powering the operation of the infusion pump unit.

In an example embodiment, the infusion fluid storage forms a part of the infusion pump device. For example, the infusion storage may be an ampoule comprising a piston that is actuated by an actuator for dosing the infusion fluid. In another example embodiment, the infusion storage device is a flexible unit such as a bag that is compressed by a desired volume with an actuator such as a piston.

In an example embodiment, the infusion pump unit 200 further comprises a user interface 255 to enable receiving input from the user and / or to enable providing information to the user. The user interface 255 may comprise any of a display; loudspeaker; speech synthesizer; touch screen; button; keypad; microphone; and speech recognizing circuitry.

In an example embodiment, the infusion pump unit 200 is configured to administer insulin. In an example embodiment, the infusion pump unit 200 is configured to administer pain medication. In an example embodiment, the infusion pump unit 200 is an ambulatory infusion pump unit. In an example embodiment, the infusion pump unit 200 is an antibiotic delivery system configured to deliver antibiotics.

In an example embodiment, the processor 235 comprises, for example, any one or more of: a master control unit (MCU); a microprocessor; a digital signal processor (DSP); an application specific integrated circuit (ASIC); a field programmable gate array; and a microcontroller. Fig. 2 presents the block diagram schematically and for ease of illustration, the fluid storage 205 is drawn as a rigid tank, whereas in a typical embodiment, the fluid storage 205 is a disposable unit, reservoir or ampoule. The fluid storage 205 can be made, for example, from plastics.

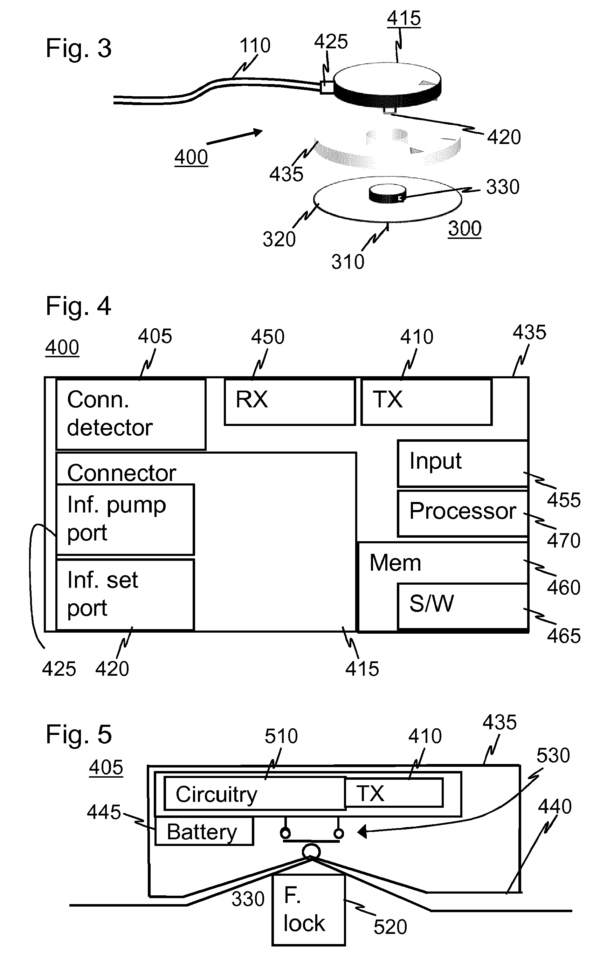

Fig. 3 shows a schematic drawing of the tubing 110, cannula set 300 and the joining unit 400 of an example embodiment. In Fig. 3, the joining unit 400 may comprise two larger parts, that is a connector 415 and an intermediate part that appears in Fig. 3 as a separate housing 435 that defines a hole through which the connector part can be locked to the cannula set 300 or to an infusion cannula 310. It should be noticed that the connector is connected to a fluid connection with the cannula 310 and to a mechanical connection to the cannula set, either through the cannula 310 or by coupling to other part(s) of the cannula set 300 that are mechanically coupled with the cannula 310. In another example embodiment, however the joining unit 400 comprises only one major part or more than two major parts. For example, in the embodiment shown by Fig. 3, a battery and electronics may be conveniently located in the intermediate part that need not be in direct contact with the infusion fluid 210.

In an example embodiments, the cannula set 300 comprises the infusion cannula 310 that is configured to deliver infusion into subcutaneous tissue of a user of the cannula set 300. In Fig. 3, the cannula set 300 comprises a skin attachment member 320 configured to maintain the cannula set 300 in place for at least a plurality of hours. In an example embodiment, the skin attachment member 320 contains adhesive material for attaching the member 320 to the skin.

In an example embodiment, the cannula set 300 comprises a connector port 330 configured to form a fluid connection with the infusion pump device 215 through the connector 415, when the connector is attached to the cannula set 300. In an example embodiment, the connector port is further configured to function as a form lock 520 (Fig. 5) that is configured to match with a corresponding form in the joining unit 400. Hence, both the joining unit 400 and the cannula 310 or cannula set 300 can be understood to comprise a form lock. The connector 415 can be provided with a cannula connection port 420 for user releasably connecting to the cannula 310 so as to arrange a fluid connection between the cannula 310 and the infusion pump device 215. The cannula set 300 may be disposable. In an example embodiment, the tubing 110 is provided for interconnecting the pump device 215 and the cannula 310. The tubing 110 may be disposable.

In an example embodiment, the connector 415 comprises an infusion pump device connection port 425 for connecting to the pump device 215 for arranging a fluid connection with the pump device 215.

Fig. 4 shows a block diagram of the joining unit 400 of an example embodiment. The joining unit 400 comprises a connection detector 405 (see also Fig. 5) configured to detect whether the infusion pump device 215 is connected to the cannula 310 with the connector 415; and a wireless transmitter 410 configured to transmit to the infusion pump unit 200 a connection indication in response to detecting by the connection detector 405 that the infusion pump device 215 is connected to the cannula 310 to form a fluid connection.

In an example embodiment, the infusion pump device connection port 425 is releasably connectable by a user to an infusion output 220 of the infusion pump device 215. The infusion pump device port 425 is connectable to the infusion output 220 via a flexible tubing, for example, such as the tubing 110. In an example embodiment, the connection port 330 comprises a form lock configured to user releasably connect the connector 415 to the cannula 310. The form lock may be configured to form a match between the cannula 310 and the connector 415. When the form lock is in a correct position or orientation (i.e. the electric circuit is closed by suitable rotation, sliding or other movement), the cannula 310 and the connector 415 are correctly aligned and in the fluid connection. Then, the infusion fluid can be pumped by the infusion pump device 215 through the cannula 310.

In an example embodiment, the housing 435 is attachable to the connector 415 to a combination that can then be connected to the cannula set 300. Alternatively or additionally, the housing 435 can be attachable to the cannula set 300 before connection of the connector 415 to the cannula set 300. For such attachment, a further form locking may be provided. Moreover, in an example embodiment, there is no form locking directly between the cannula set 300 and the connector 415, but instead these two are attached to each other by attaching the housing 435 separately to each of the cannula set 300 and the connector 415, using two different form locks, for example.

The form lock 520 comprises, for example, a bayonet base or a bayonet base socket. The form lock may comprise a thread. In an example embodiment, the connector 415 is configured to enable the connection detector 405 to detect from the connector 415 whether the connector 415 is connected to the cannula 310. In an example embodiment, the connector 415 is configured to inhibit the connection detector 405 from detecting that the connector 415 is connected to the cannula 310 unless the connector 415 is in the fluid connection with the cannula 310.

In an example embodiment, the wireless transmitter 410 comprises a radio transmitter. The wireless transmitter 410 can be a low power wireless connection means, such as the Bluetooth low energy (BLE), near field communications (NFC) - as battery powered or as passive, radio powered device, for example. Alternatively, or additionally, the wireless transmitter 410 may comprise an ultrasound transmitter, or any other suitable radio frequency (RF) transmitter.

In an example embodiment, the joining unit comprises a connection detector 405 (see Figs. 4 and 5). In an example embodiment, the connection detector is water-proof, water-resistant or hermetic. In an example embodiment, the connection detector 405 contains the wireless transmitter 410. In an example embodiment, the housing comprises a wall 440 separating the connection detector 405 from the cannula 310. The connection detector 405 may be configured to detect the connection through the wall 440. In an example embodiment, the wall 440 is flexible. In an example embodiment, the connection detector 405 is configured to detect the connection from flexing of the wall 440. In an example embodiment, the form lock 520 is configured to flex the wall 440 when the connector 415 is connected to or disconnected from the cannula 310. In an example embodiment, the housing 435 should be oriented in a particular angular orientation with regard to the connector 415 and / or the cannula set 300 so that the connection detector 405 could function properly. A particular orientation guide may be arranged, such as matching shapes that necessitate proper orientation between the housing 435 and an adjacent element selected from the connector 415 and the cannula set 300. Alternatively, the connection detection is implemented such that the housing 435 can be attached in an arbitrary angle in relation to the connector 415 and / or the cannula set 300. For example, the connection detector may be configured to detect flexing of the wall 440 around the central aperture drawn in Fig. 3.

In an example embodiment, the housing 435 contains a battery 445 for operating the wireless transmitter 410 and a circuitry 510 that is provided for controlling the operation of the connection detector 405. In an example embodiment, the connection detector 405 is configured to power the wireless transmitter 410 using the battery 445 when the connection detector 405 detects that the connector 415 is connected to the cannula 310.

The connection detector comprises a switch 530 or a sensor circuitry that is configured to detect the connection through the wall. For example, Fig. 5 schematically shows a switch 530 formed of electrically conductive surfaces that form a circuit when depressed through the wall 440. In an example embodiment, the switch is formed of conductive inner walls of the housing 435 and a conducive perimeter located adjacent to the wall and separated by a gap that is bridged by flexing of the wall when depressed by the form lock 520, for example. In other example embodiments, instead of a switch, there is provided a magnetic sensor; an inductive sensor; a capacitive sensor; an optical sensor; or any combination thereof.

The battery 445 may be disposable. The battery may be rechargeable. The battery may be integrated into the joining unit 400. In an example embodiment, the connection detector 405 is integrated to the connector 415. In case where the connection detector 405 is integrated to the connector 415, and the form lock is integrated to the cannula set connection port 330, the separate housing 435 may not be needed. The connection detector may be integrated into the housing 435. The housing 435 may be re-usable comprising a replaceable battery. The battery may be rechargeable.

Notice that the form lock 520 need not be oriented as in Fig. 5, but instead it may be vertically or diagonally oriented as well. Various bayonet type form locking mechanisms can be used, for example such as those known from electric bulbs.

In an example embodiment, the wireless transmitter 410 is configured transmit connection indications in response to detecting by the connection detector 405 that the infusion pump device 215 is connected to the cannula 310. In an example embodiment, the transmitter 410 transmits only periodically in order to save power of the battery 445. The transmitter 410 may be configured to transmit just before the joining unit 400 is used to pass next dose of the infusion fluid through the cannula 310.

In an example embodiment, the joining unit 400 comprises a receiver 450 configured to receive an infusion interval setting from the infusion pump unit 200. In an example embodiment, the infusion interval setting is used to configure the transmit interval for the transmitter 410. In an example embodiment, the receiver is a wireless receiver 450 so that the joining unit 400 can be set to transmit to the infusion pump unit 200 indications at desired rate. For example, the infusion pump unit 200 may operate by delivering set doses at set infusion rate such as once per ten seconds or by some multiple of the infusion rate, such as twice or thrice the infusion rate. Before each dose delivery, the infusion pump unit 200 may verify the fluid connection from the connection status of the connector 415. In an example embodiment, the joining unit 400 is configured to adjust the periodic transmitting of the connection indications according to a pumping interval of the infusion pump device 215.

In an example embodiment, the joining unit 400 comprises an input 455 configured to receive feedback from a sensor 120. In an example embodiment, the sensor is a Continuous Glucose Monitoring (CGM) sensor. The input 455 can be a wired or wireless input, for example the wireless receiver 450 or a wired analog or digital input. In an example embodiment, the transmitter 410 is configured to transmit the feedback (such as a sensed glucose level indication) to the infusion pump unit 200 for adjustment of infusion therapy adhered by the infusion pump unit 200. The sensor 120 may be comprised by the cannula set 300.

In an example embodiment, the joining unit 400 comprises a memory 460 including a computer program code 465. In an example embodiment, the joining unit 400 further comprises a processor 470 for controlling the operation of the joining unit 400 using the computer program code 465. The processor 470 comprises, for example, any one or more of: a master control unit (MCU); a microprocessor; a digital signal processor (DSP); an application specific integrated circuit (ASIC); a field programmable gate array; and a microcontroller.

Fig. 6 shows a flow chart of a process according to an example embodiment. The process comprises one or more of the following steps, which may be implemented using a processor or processing circuitry of the joining unit: 610. detect whether an infusion pump is connected to a cannula; 620. transmit wirelessly to the infusion pump unit a connection indication in response to detecting that the infusion pump unit is connected to the cannula; 630. receive an infusion interval setting; and 640. adjust connection indication transmission periodicity based on the infusion interval setting; and 650. alarm the user if a failure is detected in the connection of the infusion pump 215 to the cannula 310. In order to distinguish a failure state from intended disconnection, the joining unit 400 can be provided with a user input with which the user can set the joining unit 400 into an off state. The user input can be implemented using a dial, lever or other movable member that additionally operates the form lock. In an example embodiment, the user is allowed to set the joining unit between the off state and the connected state by one movement.

Without in any way limiting the scope, interpretation, or application of the claims appearing below, a technical effect of one or more of the example embodiments disclosed herein is that connection status of an infusion pump to a cannula can be automatically and robustly verified. Another technical effect of one or more of the example embodiments disclosed herein is that a user can be automatically alerted of a failure of the connection between the infusion pump and the cannula can. Yet another technical effect of one or more of the example embodiments disclosed herein is that the infusion pump unit can be informed of the connection state without need for electric wires between the infusion pump unit and the cannula set. Yet another technical effect of one or more of the example embodiments disclosed herein is that the connection detector can be made dust and moisture resistant.

Embodiments of the present invention may be implemented in software, hardware, application logic or a combination of software, hardware and application logic. In an example embodiment, the application logic, software or an instruction set is maintained on any one of various conventional computer-readable media. In the context of this document, a "computer-readable medium" may be any non-transitory media or means that can contain, store, communicate, propagate or transport the instructions for use by or in connection with an instruction execution system, joining unit, or device, such as a computer, with one example of a computer described and depicted in Fig. 4. A computer-readable medium may comprise a computer-readable storage medium that may be any media or means that can contain or store the instructions for use by or in connection with an instruction execution system, joining unit, or device, such as a computer.

If desired, the different functions discussed herein may be performed in a different order and/or concurrently with each other. Furthermore, if desired, one or more of the before-described functions may be optional or may be combined.

Although various aspects of the invention are set out in the independent claims, other aspects of the invention comprise other combinations of features from the described embodiments and/or the dependent claims with the features of the independent claims, and not solely the combinations explicitly set out in the claims.

It is also noted herein that while the foregoing describes example embodiments of the invention, these descriptions should not be viewed in a limiting sense. Rather, there are several variations and modifications which may be made without departing from the scope of the present invention as defined in the appended claims.

## Claims

1. A joining unit comprising:
a connection detector configured to detect whether an infusion pump is connected to a cannula; and
a wireless transmitter configured to transmit to the infusion pump a connection indication in response to detecting by the connection detector that the infusion pump is connected to the cannula.

2. The joining unit of claim 1, further comprising a connector configured to user releasably connect the infusion pump to the cannula into a fluid connection.

3. The joining unit of claim 2, wherein the connector is configured to enable the connection detector to detect whether the connector is connected to the cannula.

4. The joining unit of claim 2 or 3, comprising a water-proof housing;
the water-proof housing containing the connection detector.

5. The joining unit of claim 4, the water-proof housing further comprising a wall configured to be aligned between the connector and the cannula when the connector connects the infusion pump to the cannula; and
the connection detector being configured to detect the connection through the wall.

6. The joining unit of claim 5, wherein the wall is flexible and the connection detector is configured to detect the connection from flexing of the wall.

7. The joining unit of any one of the preceding claims, wherein the connection detector is configured to power the wireless transmitter using the battery only when the connection detector detects that the connector is connected to the cannula.

8. The joining unit of any one of the preceding claims, wherein the wireless transmitter is configured to transmit connection indications in response to detecting by the connection detector that the infusion pump is connected to the cannula.

9. The joining unit of claim 8, further comprising a receiver configured to receive an infusion interval setting from an infusion pump unit that comprises the infusion pump;
wherein the joining unit is configured to adjust the periodical transmission of the connection indications based on the infusion interval setting.

10. The joining unit of any one of the preceding claims, wherein the connection detector is integrated to the connector.

11. The joining unit of any of any one of the preceding claims, wherein the connection detector is integrated to a separate housing.

12. A system comprising the joining unit of the any one of the preceding claims and the infusion pump.

13. A method comprising:
detecting whether an infusion pump is connected to a cannula; and
wirelessly transmitting to the infusion pump a connection indication in response to detecting that the infusion pump is connected to the cannula.

14. The method of claim 13, further comprising:
receiving an infusion interval setting; and
adjusting a period with which subsequent connection indications are wirelessly transmitted based on the infusion interval setting.

15. The computer program product comprising computer program code stored in a non-transitory memory medium, the computer program code being configured to cause an apparatus, when executing the program code, to perform the method of claim 13 or 14.
